# EUROPEAN PATENT SPECIFICATION

(11) **EP 4 615 318 B1**
(45) Date of publication and mention of the grant of the patent: **26.08.2026**
(21) Application number: 23814247.5
(22) Date of filing: 07.11.2023
(51) Int. Cl.: A61B 5/06, A61B 5/00, A61B 1/00, A61B 34/30

(54) **MAGNETIC ROBOTIC ASSEMBLY, MAGNETIC ROBOTIC SYSTEM, METHOD FOR LOCATING A MEDICAL DEVICE**
MAGNETISCHE ROBOTERANORDNUNG, MAGNETISCHES ROBOTERSYSTEM, VERFAHREN ZUR LOKALISIERUNG EINER MEDIZINISCHEN VORRICHTUNG
ENSEMBLE ROBOTIQUE MAGNÉTIQUE, SYSTÈME ROBOTIQUE MAGNÉTIQUE, PROCÉDÉ DE LOCALISATION D'UN DISPOSITIF MÉDICAL

(30) Priority: 10.11.2022 IT 202200023214
(43) Date of publication of application: 17.09.2025
(73) Proprietor: Atlas Endoscopy Limited, Leeds LS2 3AA (GB)
(72) Inventor: SCAGLIONI, Bruno, Leeds, LS2 3AA (GB); MAZZEO, Donato, Leeds, LS2 3AA (GB); VALDASTRI, Pietro, Leeds, LS2 3AA (GB)
(74) Representative: Signori, Ludovico
(86) International application number: PCT/IB2023/061235
(87) International publication number: WO 2024/100553

(56) References cited:
- WO-A1-2021/005582
- US-A1- 2014 139 306
- US-A1- 2014 358 162
- US-A1- 2019 104 994

## Description

### . Field of the invention

**.** The present invention relates to a magnetic robotic assembly, as well as to a magnetic robotic system, as well as to a method for magnetically locating a medical device, in particular an insertion portion of a medical device.

### . Background art

**.** Magnetic robotic systems configured to locate a medical device immersed in a magnetic field are known in the art.

**.** Magnetic robotic systems are known comprising a robotic arm, in which a permanent magnet is connected to a distal end of a robotic arm, so that the permanent magnet is integrally movable at the distal end of the robotic arm by immersing the medical device in the magnetic field.

**.** The use of a magnetic robotic system provided with a single permanent magnet, due to the symmetry of the magnetic field generated by the permanent magnet, has the disadvantage of preventing the localization of the medical device in the singularities of the magnetic field, or in that plurality of points in which one or more Cartesian components of the magnetic field have the same value, preventing a unique correspondence between the value of the field detected by the medical device and the detection position.

**.** In order to solve such a drawback, US11122965 teaches to arrange an electromagnetic coil around the permanent magnet, integrally connecting it to the permanent magnet and to the distal end of the robotic arm so as to generate a sinusoidal magnetic field with a magnetic moment perpendicular to the magnetic moment of the constant magnetic field generated by the permanent magnet. The superposition of the sinusoidal magnetic field generated by the electromagnetic coil with the constant magnetic field generated by the permanent magnet prevents the magnetic field in which the medical device is immersed from having the same value in two different points.

**.** Although this solution allows uniquely locating the medical device immersed in the magnetic field, the arrangement of the coil wound around the permanent magnet generates intense vibrations in the permanent magnet when the coil is powered. The permanent magnet, vibrating intensely and at high frequency, on the one hand, generates strong noises which are unacceptable in robotic systems in the medical field, according to the regulations related to medical devices, and which are a danger to the health of users and operators with the robotic system, on the other hand it transmits vibrations in the robotic arm which can in turn be transmitted to the surfaces on which the robotic arm is supported, triggering stresses to the joints and electronics of the robotic system which can cause unwanted machine stops or accelerate wear phenomena.

**.** Therefore, the need to create a magnetic robot assembly as well as a magnetic robotic system is strongly felt in the field, which allow uniquely locating a medical device immersed in a magnetic field, reducing if not eliminating the noise and stresses to the robotic arm caused by the vibrations of the permanent magnet, while increasing the localization sensitivity, the positioning speed as compared to what is known.

### . Solution

**.** The present invention aims to provide a robotic assembly, as well as a robotic system, as well as a magnetic localization method, which allow generating a magnetic field and locating a medical device immersed in the uniquely generated and highly accurate magnetic field, reducing noise in the localization step.

**.** This and other objects and advantages are achieved with a robotic assembly according to claim **1,** as well as a robotic system according to claim **8,** as well as a localization method according to claim **9.**

**.** Some advantageous embodiments are the subject of the dependent claims.

**.** By virtue of the suggested solutions, it is possible to ensure an independent positioning of a first magnetic source configured to generate a permanent and/or constant magnetic field and a second magnetic source configured to generate a periodic and/or variable magnetic field, supported by the same robotic assembly, avoiding any vibration of a magnetic source caused by the other magnetic source, connecting the first magnetic source to a robotic arm movable in a plurality of configurations known and predetermined by the kinematics of the robotic arm, and connecting the second magnetic source to a robot base supporting the robotic arm, allowing the relative position between the first magnetic source and the second magnetic source to be simultaneously known for each configuration in which the robotic arm is movable.

**.** By virtue of the suggested solutions, it is possible to ensure an independence of the dimensions of the first magnetic source from the dimensions of the second magnetic source and vice versa, allowing the creation of robotic assemblies and robotic systems which can generate magnetic fields which are easily adaptable depending on the conditions in which they must operate.

**.** By virtue of the suggested solutions, it is possible to avoid a connection with the robotic arm of the magnetic source configured to generate a periodic and/or variable magnetic field, avoiding having an electrical connection through the robotic arm to power the second magnetic source, which instead, being constrained to the robot base, can be easily powered without power constraints.

**.** By virtue of the suggested solutions, it is possible to reduce the masses supported by the robotic arm as compared to what is known, while allowing a unique localization of the medical device immersed in the magnetic field, as well as a faster and more accurate positioning speed of the first magnetic source as compared to what is known.

### . Figures

**.** Further features and advantages of the magnetic robotic assembly, the magnetic robotic system, and the localization method will become apparent from the description below of preferred embodiments thereof, provided by way of non-limiting indication, with reference to the accompanying drawings, in which:

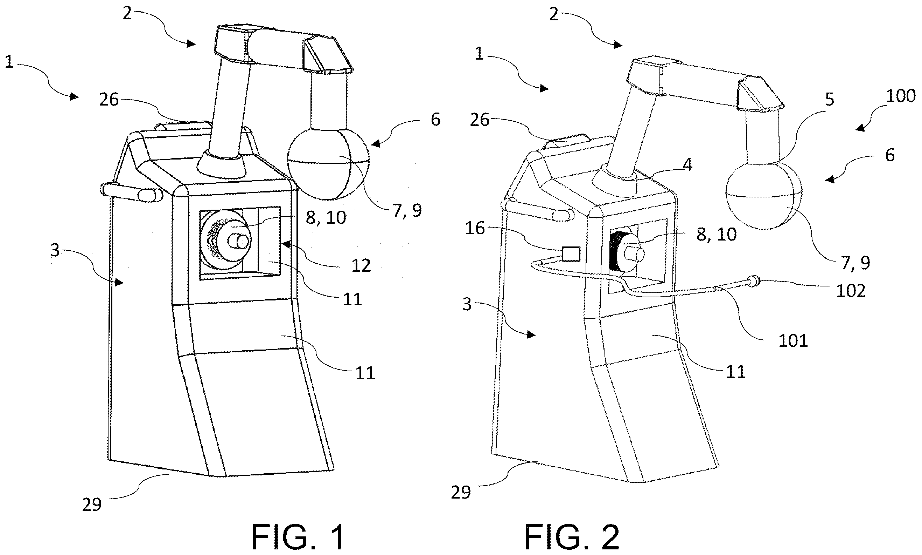

- figure 1 depicts an axonometric view of a magnetic robotic assembly according to the present invention, in which a first magnetic source can be seen, which is for generating a permanent and/or constant magnetic field directly connected to the distal end of a robotic arm to be integrally movable with the robotic arm, as an effector of the magnetic robotic assembly, and a second magnetic source supported by a robot base in a fixed position with respect to any position in which said first magnetic source can be arranged, in which the second magnetic source is configured to generate a periodic and/or variable magnetic field;
- figure 2 depicts an axonometric view of a magnetic robotic system according to the present invention, comprising the magnetic robotic assembly in figure 1 and a medical device operatively connected to said magnetic robotic assembly, preferably an endoscope provided with an insertion portion adapted to be inserted into a cavity, and preferably connected to a flexible body connected to the robot base;

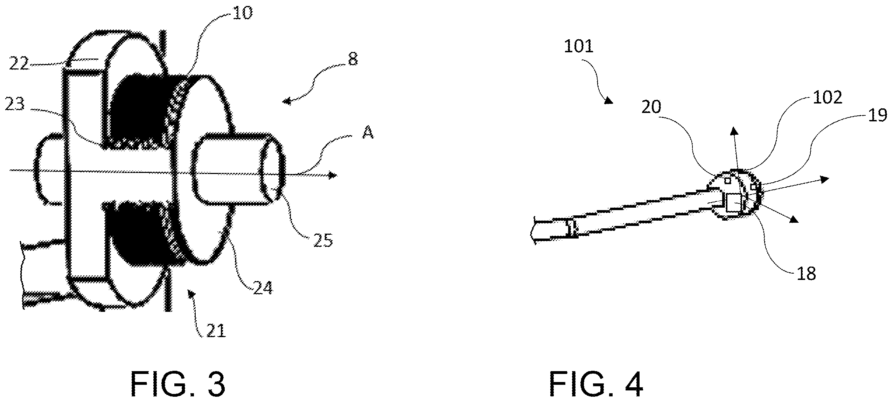

- figure 3 depicts a partially sectioned axonometric view of the second magnetic source of the robot assembly in figures 1 and 2;
- figure 4 shows an axonometric view of the insertion portion of the medical device to be located, in which a plurality of sensors supported by the insertion portion for measuring accelerations, angular velocities and magnetic field values along at least three device axes orthogonal and/or incident to each other are shown;

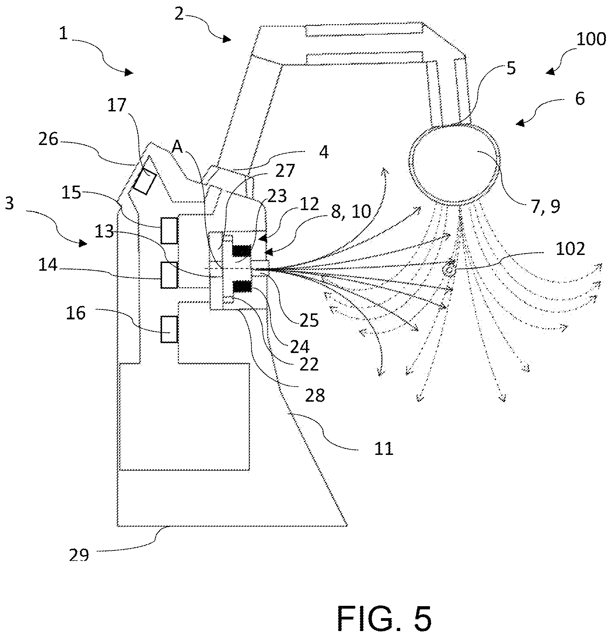

- figure 5 depicts a side view of the magnetic robotic system in figure 2 partially sectioned, in which the lines of the permanent and/or constant magnetic field generated by the first magnetic source and the lines of the periodic and/or variable and/or periodically variable magnetic field generated by the second magnetic source are visible, in which the lines of the magnetic field generated by the first magnetic source and the lines of the magnetic field generated by the second magnetic source are incidental and/or perpendicular, avoiding any parallelism, in which the insertion portion of the medical device immersed in the lines of the magnetic field generated by the first magnetic source and in the lines of the magnetic field generated by the second magnetic source can be seen;

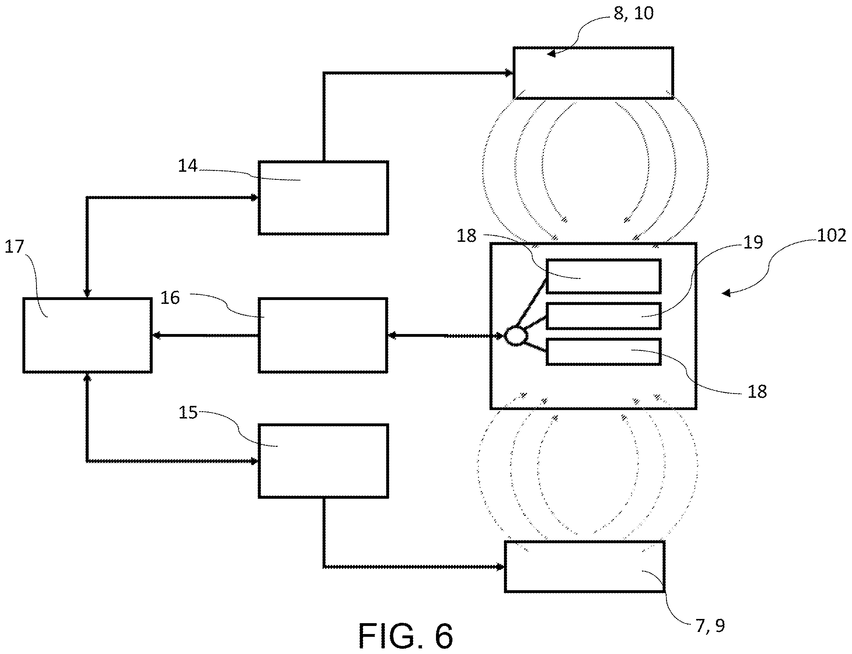

- figure 6 diagrammatically depicts the localization method implemented in the robotic system in figure 5.

### . Description of some preferred embodiments

**.** In accordance with a general embodiment, a magnetic robotic assembly for magnetically locating a medical device 101 is indicated by reference numeral 1 as a whole.

**.** The magnetic robotic assembly 1 comprises a robotic arm 2 and a robot base 3 configured to support the robotic arm 2. The robotic arm 2 is movable in a plurality of configurations with respect to said robot base 3.

**.** The robotic arm 2 extends at least between a robotic arm basal end 4 and a robotic arm distal end 5, in which said robotic arm basal end 4 is connected to said robot base 3.

**.** The magnetic robotic assembly 1 comprises a first magnetic source 7 configured to generate a first magnetic field, in which the first magnetic field is constant.

**.** The first magnetic source 7 is connected to said robotic arm distal end 5. The first magnetic source 7 is movable integrally with said robotic arm distal end 2 in a plurality of operating positions of the first magnetic source with respect to said robot base 3 so as to immerse at least one insertion portion 102 of the medical device 101 in the first magnetic field by moving said robotic arm 5.

**.** The magnetic robotic assembly 1 comprises at least a second magnetic source 8 configured to generate a second magnetic field, in which the second magnetic field is periodically variable.

**.** Advantageously, said at least a second magnetic source 8 is constrained to said robot base 3 so as to be arranged in a second magnetic source position which is fixed and integral with respect to said robot base 3 for each first magnetic source position of said first magnetic source 7.

**.** By virtue of the positioning of the at least a second magnetic source 8 in a fixed and integral position with the robot base 3 and of the first magnetic source 7 in a position integral with the robotic arm distal end 5, it is possible to provide a magnetic robotic assembly having two magnetic sources positioned, one movable and one fixed, in positions independent of each other, in which it is possible to know a priori, the relative position between the first magnetic source 7, movable, with respect to the at least a second magnetic source 8, the kinematics with which the robotic arm 2 moves in space being known.

**.** By virtue of the positioning of the at least a second magnetic source 8 in a fixed and integral position with the robot base 3, it is possible to avoid or at least reduce a magnetic interaction between the second magnetic field and the first magnetic source 7, avoiding causing unwanted vibrations in the first magnetic source 7 and therefore in the robotic arm 2.

**.** In accordance with an embodiment, said at least a second magnetic source 8 is oriented so that when said first magnetic source 7 is in any first magnetic source operating position of said plurality of first magnetic source operating positions, the second magnetic field is superimposed on the first magnetic field.

**.** In accordance with an embodiment, said at least a second magnetic source 8 is oriented so as to immerse at least the insertion portion 102 in the first magnetic field and in the second magnetic field.

**.** In accordance with an embodiment, said at least a second magnetic source 8 is oriented so as to avoid a parallelism between the field lines and/or the magnetic field vectors of said second magnetic field and the field lines and/or the magnetic field vectors of said first magnetic field in any position in which the first magnetic field is superimposed on the second magnetic field.

. In accordance with an embodiment, said first magnetic source 7 is connected to said robotic arm distal end 5, avoiding electrical connections for electrically powering said first magnetic source 7 between said first magnetic source 7 and said robotic arm 2 and/or said robot base 3. High power electrical connections supported by the robotic arm 2 can thus be avoided.

**.** In accordance with an embodiment, said magnetic robotic assembly 1 comprises an effector 6 directly connected to said robotic arm distal end 5, in which said effector 6 comprises said first magnetic source 7 avoiding supporting further magnetic sources configured to generate a respective further magnetic field.

**.** In accordance with an embodiment, said first magnetic source 7 is a permanent magnet 9 configured to generate said first magnetic field.

**.** In accordance with an embodiment, said at least one magnetic source 8 is an electromagnet configured to generate said second magnetic field.

**.** In accordance with an embodiment, said at least a second magnetic source 8 comprises at least one winding 10 configured to generate said second magnetic field when crossed by a current.

**.** In accordance with an embodiment, said robot base 3 comprises a base body 11 delimiting at least one seat 12 of second magnetic source, in which said at least a second magnetic source 8 is housed in said seat 12 of second magnetic source in connection with said robot base 3.

**.** In accordance with an embodiment, said at least one magnetic source 8 is electrically powered by an electrical connection of a second magnetic source, in which said electrical connection is housed inside said base body 11. It is thus possible to avoid the presence of electrical connections of second magnetic source reachable outside the robot base 3, and/or which can be hindering in a zone in which the robot assembly 1 operates and/or can form an electrical hazard for an operator of the robot assembly or for a patient on whom the robot assembly operates.

**.** In accordance with an embodiment, said robot base 3 comprises a support portion 13 adapted to directly support said at least a second magnetic source 8. In accordance with an embodiment, said support portion 13 at least partially delimits said at least a seat 12 of second magnetic source.

**.** In accordance with an embodiment, said robot base 3 comprises reversible connection means 13 configured to reversibly connect and/or form a reversible coupling between said at least a second magnetic source 8 and said robot base 3. In accordance with an embodiment, the reversible coupling between the robot base 3 and the second magnetic source is a reversible shape coupling between a portion of the second magnetic source and a portion of the robot base. In accordance with an embodiment, said reversible connection means 13 are coupling means configured to couple the second magnetic source to the robot base 3.

**.** In accordance with an embodiment, the reversible coupling between said at least a second magnetic source 8 and said robot base 3 allows replacing the second magnetic source 8 connected to the robot base with another second magnetic source 8. In accordance with an embodiment, a robotic assembly kit comprises a robotic assembly according to one of the embodiments described herein and a set of second magnetic sources, in which each second magnetic source is reversibly connectable to the robot base 3 and is replaceable with another second magnetic source of the set of magnetic sources.

**.** In accordance with an embodiment, said at least a second magnetic source 8 comprises a coil support 21 adapted to support said at least one winding 10, in which said coil support 21 has at least one hollow portion to electrically connect the at least one winding 10.

**.** In accordance with an embodiment, the coil support 21 comprises at least one bottom plate 22 connected to a winding portion 23, preferably tubular, in which said at least one winding 10 is wound around said winding portion 23. In accordance with an embodiment, said coil support 21 comprises a front plate 24 adapted to couple to the winding portion 23 on an opposite side with respect to the bottom plate 22.

**.** In accordance with an embodiment, the at least a second magnetic source 8 comprises a magnetic core 25 so as to concentrate the lines of the second magnetic field in the material of the magnetic core 25. In accordance with an embodiment, the magnetic core 25 is coaxial with said winding portion 23 and/or with said winding. In accordance with an embodiment, the magnetic core 25 is connected to said front plate 24 at least on the opposite side to said winding portion 23.

**.** In accordance with an embodiment, said robot base 3 comprises a control panel 26 adapted to control said magnetic robotic assembly 1. In accordance with an embodiment, said magnetic robotic assembly 1 defines a front zone in which the robotic arm distal end 5 is mainly adapted to move. In accordance with an embodiment, the control panel 26 is arranged on the base body 11 in a zone opposite to the front zone.

**.** In accordance with an embodiment, said base body 4 avoids shielding the second magnetic field at least towards said front part of the magnetic robotic assembly 1.

**.** In accordance with an embodiment, said base body 11 shields the second magnetic field at least towards the control panel.

**.** In accordance with an embodiment, said base body 4 comprises at least one seat bottom wall 27 and at least one seat side wall 28 delimiting said at least one seat 12 of second magnetic source.

**.** In accordance with an embodiment, said at least one seat side wall 28 delimits a seat opening 29 with an edge thereof. In accordance with an embodiment, said seat opening 29 is opposite to said at least one seat bottom wall 27 allowing an access to said at least one seat 12 of second magnetic source from an environment outside the base body 4, for example from said front zone.

**.** In accordance with an embodiment, said at least one seat bottom wall 27 and said at least one seat side wall 28 are connected so as to define a box-like shape, for example cylindrical or prismatic.

**.** In accordance with an embodiment, said at least one seat bottom wall 27 and said at least one seat side wall 28 are configured to shield the second magnetic field generated by said at least a second magnetic source 8 avoiding shielding in the direction of said seat opening 29.

**.** In accordance with an embodiment, said at least one winding 10 defines a winding axis A. In accordance with an embodiment, said robot base 3 comprises a pedestal 29 adapted to rest on a support surface or a floor to stably support the robotic arm 2.

**.** In accordance with an embodiment, the pedestal 29 comprises an abutment plane, in which the winding axis A is parallel to the abutment plane.

**.** In accordance with an embodiment, the winding axis A forms with the abutment plane of said pedestal 29 a winding angle between 0 and 60 degrees, preferably between 0 and 45 degrees.

**.** In accordance with an embodiment, said robotic arm 2 comprises at least one plurality of rigid links or connections, and a plurality of rotational joints which allow a rotation of one link with respect to another with a rotational degree of freedom. In accordance with an embodiment, said plurality of rigid links or connections comprises at least one distal link and one basal link. In accordance with an embodiment, said distal link comprises said robotic arm distal end 5. In accordance with an embodiment, said basal link comprises said robotic arm basal end 4.

**.** The present invention further relates to a magnetic robotic system 100 for magnetically locating a medical device 101. The magnetic robotic system 100 comprises at least one magnetic robotic assembly 1 according to any of the described embodiments.

**.** The magnetic robotic system 100 comprises at least said medical device 101, in which said medical device 101 comprises an insertion portion 102 adapted to be introduced into a cavity.

**.** The insertion portion 102 comprises a plurality of magnetic field sensors 18 configured to detect a three-dimensional magnetic field vector and/or at least three magnetic field components with respect to at least three mutually incident axes of a magnetic field in which said insertion portion 102 is immersed. In accordance with an embodiment, the plurality of magnetic field sensors 18 is configured to detect magnetic field signals. In accordance with an embodiment, said insertion portion 102 defines at least three first device axes, in which said at least three first device axes are incidental and/or orthogonal to each other. In accordance with an embodiment, said plurality of magnetic field sensors 18 is at least three magnetic field sensors configured to each detect a respective component of the three-dimensional magnetic field vector with respect to a respective first device axis. In accordance with an embodiment, said plurality of magnetic field sensors 18 comprises at least one Hall-effect sensor.

**.** The insertion portion 102 comprises a triaxial accelerometer 19 configured to detect a three-dimensional vector of acceleration and/or at least three accelerations with respect to three mutually incident axes of said insertion portion 102. In accordance with an embodiment, said device insertion portion 102 defines at least three second device axes, in which the at least three second device axes are mutually incidental and/or orthogonal. In accordance with an embodiment, said triaxial accelerometer 19 is configured to detect each component of said three-dimensional acceleration vector with respect to each of said at least three second device axes.

**.** The insertion portion 102 comprises a triaxial gyroscope 20 configured to detect at least three angular velocities of said insertion portion 102 with respect to at least three mutually incident and/or orthogonal axes. In accordance with an embodiment, said device insertion portion 102 defines at least three third device axes, in which the at least three third device axes are mutually incidental and/or orthogonal. In accordance with an embodiment, said triaxial gyroscope 20 is configured to detect an angular velocity with respect to each of said at least three device axes.

**.** The magnetic robotic system 100 comprises a first magnetic source position control unit 15 configured to detect the position of the first magnetic source between said plurality of first magnetic source positions of the first magnetic source 7.

**.** The magnetic robotic system 100 comprises a second magnetic field control unit 14 configured to control a predefined frequency and a predefined periodic waveform to generate said second magnetic field with said at least a second magnetic source 8. In accordance with an embodiment, said predefined periodic waveform is a sine wave and/or square wave and/or triangular wave and/or sawtooth wave. In accordance with an embodiment, said predefined periodic waveform has said predefined frequency. In accordance with an embodiment, said predefined frequency is at least 200 Hz.

**.** The magnetic robotic system 100 comprises a processing unit 17 configured to calculate and/or estimate a roll and a pitch of said insertion portion 102 with respect to a reference point of said robot base 4 based on said three-dimensional acceleration vector and said at least three angular velocities detected by said triaxial accelerometer 19 and said triaxial gyroscope 20, respectively.

**.** Advantageously, said processing unit 17 is configured to calculate and/or estimate a medical device position Xp, Yp, Zp of said insertion portion 102 and a yaw of said insertion portion 102 with respect to said reference point of said robot base 4 based on the calculated roll, the calculated pitch, the detected three-dimensional magnetic field vector, a relative position of magnetic sources defined by the difference between the detected first magnetic source position and the second magnetic source position fixed and integral with the robot base 4, a relative position of second magnetic source defined by the difference between the second magnetic source position fixed and integral with the robot base 4 and the reference point of said robot base 4, from the predefined frequency and the predefined periodic waveform.

**.** In accordance with an embodiment, said first magnetic source position control unit 15, said second magnetic field control unit 14, and said processing unit 17 are housed in a housing obtained in said robot base 3. In accordance with an embodiment, said first magnetic source position control unit 15 is operatively connected to said robotic arm 2. In accordance with an embodiment, the second magnetic field control unit 14 is operatively connected to said at least a second magnetic source 8.

**.** In accordance with an embodiment, said medical device 101 is an endoscope directly connected to said robotic assembly 1, and in which said insertion portion 102 is the tip of the endoscope, in which said insertion portion 102 is connected to the robotic assembly 1 by means of a flexible endoscope body or shaft.

**.** In accordance with an embodiment, the magnetic robotic system 100 comprises a data transceiver unit 16 configured to receive at a detection frequency said three-dimensional magnetic field vector, said three-dimensional acceleration vector and said at least three angular velocities, from said plurality of magnetic field sensors 18, from said triaxial accelerometer 19 and from said triaxial gyroscope 20, respectively; in which said data transceiver unit 16 is configured to transmit said detected three-dimensional magnetic field vector, said detected three-dimensional acceleration vector and said at least three angular velocities, to said processing unit 17.

**.** In accordance with an embodiment, said processing unit 17 comprises a memory unit for storing one or more of: said detected three-dimensional magnetic field vector, said detected three-dimensional acceleration vector, said at least three detected angular velocities, said calculated roll, said calculated pitch, said relative position of magnetic sources, said relative position of second magnetic source, said reference point of said robot base 4, said predefined frequency and said predefined waveform.

**.** In accordance with an embodiment, said processing unit 17 is operatively connected to said effector position control unit 15 for acquiring, storing, and calculating an average of the relative position between said first magnetic source 7 and said at least a second magnetic source 8.

**.** In accordance with an embodiment, said processing unit 17 is operatively connected to said data transceiver unit 16 for acquiring, storing, and calculating a first function of the detected three-dimensional acceleration vectors, a second function of the detected three-dimensional angular velocity vectors, and an average of the detected three-dimensional magnetic field vectors. In accordance with an embodiment, said first function and/or said second function are a mathematical function such as a mean, a median or a function referring to the last detected sample.

**.** In accordance with an embodiment, said processing unit 17 is operatively connected to said second magnetic field control unit 16 for acquiring and storing the waveform of the second magnetic field, and for calculating amplitude and phase of the detected magnetic field.

**.** The present invention further relates to a method for locating a medical device 101 immersed in a magnetic field. The medical device 101 comprises an insertion portion 102 adapted to be introduced into a cavity. Said insertion portion 102 comprises a plurality of magnetic field sensors 18 configured to detect a three-dimensional magnetic field vector of said magnetic field in which said insertion portion 102 is immersed. Said insertion portion 102 comprises a triaxial accelerometer 19 configured to detect a three-dimensional vector of acceleration of said insertion portion 102. Said insertion portion 102 comprises a triaxial gyroscope 20 configured to detect at least three angular velocities of said insertion portion 102 with respect to at least three mutually incident axes.

**.** The method comprises the steps of:
- providing a robot assembly 1 according to any of the described embodiments;
- generating, by said first magnetic source 7 and said second magnetic source 8, said first magnetic field and said second magnetic field, respectively, so that the medical device 101 is immersed at least in the superposition of the first magnetic field and the second magnetic field,
- keeping the first magnetic field constant,
- periodically varying the second magnetic field with a predefined periodic wave function and a predefined frequency,
- detecting the position of the second magnetic source fixed and integral with the robot base 4,
- defining a reference position of said robot base 4,
- calculating a relative position of second magnetic source defined by the difference between the second magnetic source position and the reference position,
- detecting, acquiring and storing the first magnetic source position of the first magnetic source 7,
- calculating a relative position of magnetic sources defined by the difference between the detected first magnetic source position and the second magnetic source position fixed and integral with the robot base 4,
- detecting, acquiring and storing at an acquisition frequency said three-dimensional magnetic field vector, said three-dimensional acceleration vector and said at least three angular velocities,
- calculating and storing a roll and a pitch of said insertion portion 102 with respect to a reference point of said robot base 4 based on said three-dimensional acceleration vector and said at least three angular velocities detected by said triaxial accelerometer 19 and by said triaxial gyroscope 20, respectively,
- calculating a medical device position Xp, Yp, Zp of said insertion portion 102 and a yaw of said insertion portion 102 with respect to said reference point of said robot base 4 based on the calculated roll, the calculated pitch, the calculated relative position of magnetic sources, the calculated relative position of second magnetic source, the three-dimensional magnetic field vector, the predefined periodic wave function, and the predefined frequency.

**.** In accordance with an operating mode, the method comprises the step of providing a robot system 100 according to any one of the described embodiments.

**.** In accordance with an operating mode, the method comprises the step of - calculating and storing at a calculation frequency a first function, for example an average or median or last value detected, of the three-dimensional acceleration vector detected and/or the Cartesian components thereof detected at said acquisition frequency, a second function, for example an average or median or last value detected, of said at least three angular velocities detected at said acquisition frequency, and an average of the three-dimensional magnetic field vector detected and/or the Cartesian components thereof detected at said acquisition frequency, in which the average of the three-dimensional magnetic field vector detected at said acquisition frequency is significant of the first magnetic field detected.

**.** In accordance with an operating mode, the method comprises the step of calculating and storing at said calculation frequency an average of the first magnetic source position of the first magnetic source 7 as an average between the first magnetic source positions detected.

**.** In accordance with an operating mode, the method comprises the step of calculating and storing at said calculation frequency the phase and amplitude of the detected magnetic field associated with the second magnetic field source 8 based on a sequence of three-dimensional magnetic field vectors detected by the plurality of magnetic field sensors 18 in a time interval between an amplitude and phase calculation and the next, in which the magnetic field phase and amplitude calculated at the calculation frequency are significant of the second magnetic field.

**.** In accordance with an operating mode, said roll and said pitch of said insertion portion 102 are estimated based on the first function of the three-dimensional acceleration vector, and the second function of the at least three angular velocities detected.

**.** In accordance with an operating mode, said medical device position Xp, Yp, Zp and said yaw are estimated based on said roll calculated at said calculation frequency, said pitch calculated at said calculation frequency, said average magnetic field calculated at said calculation frequency, the magnetic field amplitude and phase calculated, the average of the first magnetic source position, on models of magnetic field generated by said first magnetic source 7 and said second magnetic source 8.

**.** In accordance with an operating mode, said acquisition frequency is at least one order of magnitude, preferably two orders of magnitude, higher than said calculation frequency. In accordance with an operating mode, said acquisition frequency is at least 20 kHz, and said calculation frequency is at least 100 Hz.

**.** In accordance with an operating mode, the phase and amplitude of the magnetic field detected are calculated by variants of the fast Fourier transforms, such as the Goertzel algorithm, the predefined frequency and the predefined periodic wave function being known.

**.** In accordance with an operating mode, said roll and said pitch of said insertion portion 102 are estimated through finite and infinite response filters, such as a Mahony filter.

**.** In accordance with an operating mode, said magnetic field models generated by said first magnetic source 7 and said second magnetic source 8 used for the estimation of said medical device position Xp, Yp, Zp and said yaw are a magnetic dipole model and/or generalized elliptical integral models.

**.** In accordance with an operating mode, for the estimation of said medical device position Xp, Yp, Zp and said yaw, statistical observatories are used, in which said statistical observatories comprise pseudo-Montecarlo-type algorithms, such as a particle filter, or other statistical filters, such as a Kalmann filter.

### LIST OF REFERENCE SIGNS

- 1: robotic magnetic assembly
- 2: robotic arm
- 3: robot base
- 4: robotic arm basal end
- 5: robotic arm distal end
- 6: effector
- 7: first magnetic source
- 8: second magnetic source
- 9: permanent magnet
- 10: winding
- 11: base body
- 12: seat of second magnetic source
- 13: support portion
- 14: second magnetic field control unit
- 15: first magnetic source position control unit
- 16: data transceiver unit
- 17: processing unit
- 18: plurality of magnetic field sensors or Hall-effect sensors
- 19: triaxial accelerometer
- 20: triaxial gyroscope
- 21: coil support
- 22: bottom plate
- 23: winding portion
- 24: front plate
- 25: magnetic core
- 26: control panel
- 27: seat bottom wall
- 28: seat side wall
- 29: pedestal
- 100: robotic magnetic system
- 101: medical device
- 102: insertion portion

- A: winding axis

## Claims

1. A magnetic robotic assembly (1) for magnetically locating a medical device (101), comprising:
- a robotic arm (2),
- a robot base (3) configured to support the robotic arm (2), wherein the robotic arm (2) is movable in a plurality of configurations with respect to said robot base (3),
wherein the robotic arm (2) extends at least between a robotic arm basal end (4) and a robotic arm distal end (5), wherein said robotic arm basal end (4) is connected to said robot base (3);
- a first magnetic source (7) configured to generate a first magnetic field and at least a second magnetic source (8) configured to generate a second magnetic field, wherein the first magnetic field is constant and wherein the second magnetic field is periodically variable,
wherein the first magnetic source (7) is connected to said robotic arm distal end (5), wherein the first magnetic source (7) is movable integrally with said robotic arm distal end (5) in a plurality of operating positions of the first magnetic source with respect to said robot base (3) so as to immerse at least one insertion portion (102) of the medical device (101) in the first magnetic field by moving said robotic arm (2);
**characterized in that**
said at least a second magnetic source (8) is constrained to said robot base (3) so as to be arranged in a second magnetic source position which is fixed and integral with respect to said robot base (3) for each first magnetic source position of said first magnetic source (7).

2. A magnetic robotic assembly (1) according to the preceding claim, wherein said at least a second magnetic source (8) is oriented so that when said first magnetic source (7) is in any first magnetic source operating position of said plurality of first magnetic source operating positions, the second magnetic field is superimposed on the first magnetic field so as to immerse at least the insertion portion (102) in the first magnetic field and the second magnetic field, and/or so as to avoid a parallelism between the magnetic field vectors of said second magnetic field and the magnetic field vectors of said first magnetic field in any position in which the first magnetic field is superimposed on the second magnetic field.

3. A magnetic robotic assembly (1) according to any one of the preceding claims, comprising one or more of the following features or a combination thereof:
- wherein said magnetic robotic assembly (1) comprises an effector (6) directly connected to said robotic arm distal end (5), wherein said effector (6) comprises said first magnetic source (7) avoiding supporting further magnetic sources configured to generate a respective further magnetic field;
and/or wherein said first magnetic source (7) is a permanent magnet (9) configured to generate said first magnetic field.

4. A magnetic robotic assembly (1) according to any one of the preceding claims, comprising one or more of the following features or a combination thereof:
- wherein said at least a second magnetic source (8) comprises at least one winding (10) configured to generate said second magnetic field when crossed by a current, and/or
- wherein said at least one magnetic source (8) is an electromagnet configured to generate said second magnetic field, and/or
- wherein said robot base (3) comprises a base body (11) delimiting at least one seat (12) of second magnetic source, wherein said at least a second magnetic source (8) is housed in said seat (12) of second magnetic source in connection with said robot base (3), and wherein said at least one magnetic source (8) is electrically powered by an electrical connection of a second magnetic source, wherein said electrical connection is housed inside said base body (11).

5. A magnetic robotic assembly (1) according to the preceding claim, comprising one or more of the following features or a combination thereof:
- wherein said robot base (3) comprises a support portion (13) adapted to directly support said at least a second magnetic source (8), wherein said support portion (13) at least partially delimits said at least one seat (12) of second magnetic source; and/or
- wherein said robot base (3) comprises reversible connection means (13) configured to reversibly connect said at least a second magnetic source (8) to said robot base (3) allowing a replacement of said at least a second magnetic source (8) with another from a set of second magnetic sources; and/or
- wherein said at least a second magnetic source (8) comprises a coil support (21) adapted to support said at least one winding (10), wherein said coil support (21) has at least one hollow portion to electrically connect the at least one winding (10), and/or
- wherein the at least a second magnetic source (8) comprises a magnetic core (25) so as to concentrate the lines of the second magnetic field in the material of the magnetic core (25).

6. A magnetic robotic assembly (1) according to any one of claims **4** to **5,** wherein said robot base (3) comprises a control panel (26) adapted to control said magnetic robotic assembly (1),
wherein said magnetic robotic assembly (1) defines a front zone in which the robotic arm distal end (5) is mainly adapted to move, wherein the control panel (26) is arranged on the base body (11) in a zone opposite to the front zone,
wherein said base body (4) avoids shielding the second magnetic field at least towards said front part of the magnetic robotic assembly (1),
and wherein said base body (11) shields the second magnetic field at least towards the control panel.

7. A magnetic robotic assembly (1) according to any one of claims **4** to **6,** wherein said at least one winding (10) defines a winding axis (A), and wherein said robot base (3) comprises a pedestal (29) adapted to rest on a support surface or a floor to stably support the robotic arm (2), wherein the pedestal (29) comprises an abutment surface, wherein the winding axis (A) is parallel to the abutment surface or wherein the winding axis (A) forms a winding angle between 0 and 60 degrees with the abutment surface of said pedestal (29).

8. A magnetic robotic system (100) for magnetically locating a medical device (101), comprising:
- a magnetic robotic assembly (1) according to any one of the preceding claims;
- said medical device (101), wherein said medical device (101) comprises an insertion portion (102) adapted to be introduced into a cavity,
wherein said insertion portion (102) comprises a plurality of magnetic field sensors (18) configured to detect a three-dimensional magnetic field vector of a magnetic field in which said insertion portion (102) is immersed,
wherein said insertion portion (102) comprises a triaxial accelerometer (19) configured to detect a three-dimensional vector of three-dimensional acceleration of said insertion portion (102), wherein said insertion portion (102) comprises a triaxial gyroscope (20) configured to detect at least three angular velocities of said insertion portion (102) with respect to three mutually incident and/or orthogonal axes;
- a first magnetic source position control unit (15) configured to detect the position of the first magnetic source between said plurality of first magnetic source positions of the first magnetic source (7),
- a second magnetic field control unit (14) configured to control a predefined frequency and a predefined waveform to generate said second magnetic field with said at least a second magnetic source (8);
- a processing unit (17) configured to calculate and/or estimate a roll and a pitch of said insertion portion (102) with respect to a reference point of said robot base (4) based on said three-dimensional acceleration vector and said at least three angular velocities detected by said triaxial accelerometer (19) and said triaxial gyroscope (20), respectively,
wherein said processing unit (17) is configured to calculate and/or estimate a medical device position (Xp, Yp, Zp) of said insertion portion (102) and a yaw of said insertion portion (102) with respect to said reference point of said robot base (4) based on the calculated roll, the calculated pitch, the detected three-dimensional magnetic field vector, a relative position of magnetic sources defined by the difference between the detected first magnetic source position and the second magnetic source position fixed and integral with the robot base (4), a relative position of second magnetic source defined by the difference between the second magnetic source position fixed and integral with the robot base (4) and the reference point of said robot base (4), from the predefined frequency and the predefined waveform.

9. A method for locating a medical device (101) immersed in a magnetic field, wherein said medical device (101) comprises an insertion portion (102) adapted to be introduced into a cavity, wherein said insertion portion (102) comprises a plurality of magnetic field sensors (18) configured to detect said magnetic field in which said insertion portion (102) is immersed,
a triaxial accelerometer (19) configured to detect a three-dimensional acceleration vector of said insertion portion (102),
a triaxial gyroscope (20) configured to detect at least three angular velocities of said insertion portion (102) with respect to three mutually incident and/or orthogonal axes;
wherein said method comprises the steps of:
- providing a robot assembly (1) according to any one of claims **1** to **7,**
- generating, by said first magnetic source (7) and said second magnetic source (8), said first magnetic field and said second magnetic field, respectively, so that the medical device (101) is immersed at least in the superposition of the first magnetic field and the second magnetic field,
- keeping the first magnetic field constant,
- periodically varying the second magnetic field with a predefined periodic wave function and a predefined frequency,
- detecting the position of the second magnetic source fixed and integral with the robot base (4),
- defining a reference position of said robot base (4),
- calculating a relative position of second magnetic source defined by the difference between the second magnetic source position and the reference position,
- detecting, acquiring and storing the first magnetic source position of the first magnetic source (7),
- calculating a relative position of magnetic sources defined by the difference between the detected first magnetic source position and the second magnetic source position fixed and integral with the robot base (4),
- detecting, acquiring and storing at an acquisition frequency said three-dimensional magnetic field vector, said three-dimensional acceleration vector and said at least three angular velocities,
- calculating and storing a roll and a pitch of said insertion portion (102) with respect to a reference point of said robot base (4) based on said three-dimensional acceleration vector and said at least three angular velocities detected by said triaxial accelerometer (19) and said triaxial gyroscope (20), respectively,
- calculating a medical device position (Xp, Yp, Zp) of said insertion portion (102) and a yaw of said insertion portion (102) with respect to said reference point of said robot base (4) based on the calculated roll, the calculated pitch, the calculated relative position of magnetic sources, the calculated relative position of second magnetic source, the three-dimensional magnetic field vector, the predefined periodic wave function, and the predefined frequency.

10. A method according to the preceding claim, comprising the further steps of:
- calculating and storing at a calculation frequency a first function, for example an average or median or last value detected, of the three-dimensional acceleration vectors detected and/or the Cartesian components thereof detected at said acquisition frequency, a second function, for example an average or median or last value detected, of the three-dimensional angular velocity vectors detected and/or the Cartesian components thereof detected at said acquisition frequency, and an average of the three-dimensional magnetic field vectors detected and/or the Cartesian components thereof detected at said acquisition frequency, wherein the average of the three-dimensional magnetic field vector detected at said acquisition frequency is significant of the first magnetic field detected,
- calculating and storing at said calculation frequency an average of the first magnetic source position of the first magnetic source (7) as an average between the first magnetic source positions detected,
- calculating and storing at said calculation frequency the phase and amplitude of the detected magnetic field associated with the second magnetic field source (8) based on a sequence of three-dimensional magnetic field vectors detected by the plurality of magnetic field sensors (18) in a time interval between an amplitude and phase calculation and the next, wherein the magnetic field phase and amplitude calculated at the calculation frequency are significant of the second magnetic field,
wherein said roll and a pitch of said insertion portion (102) are estimated based on the first function of the three-dimensional acceleration vector and the second function of said at least three angular speeds detected;
wherein said medical device position (Xp, Yp, Zp) and said yaw are estimated based on said roll calculated at said calculation frequency, said pitch calculated at said calculation frequency, said average magnetic field calculated at said calculation frequency, the magnetic field amplitude and phase calculated, the average of the first magnetic source position, on models of magnetic field generated by said first magnetic source (7) and said second magnetic source (8).

11. A method according to the preceding claim,
wherein said acquisition frequency is at least one order of magnitude, preferably two orders of magnitude, higher than said calculation frequency.

## Patentansprüche

1. Magnetische Roboteranordnung (1) zur magnetischen Lokalisierung einer medizinischen Vorrichtung (101), umfassend:
- einen Roboterarm (2),
- eine Roboterbasis (3), die dazu konfiguriert ist, den Roboterarm (2) zu stützen, wobei der Roboterarm (2) in einer Vielzahl von Konfigurationen in Bezug auf die Roboterbasis (3) bewegbar ist, wobei sich der Roboterarm (2) zumindest zwischen einem basalen Roboterarmende (4) und einem distalen Roboterarmende (5) erstreckt, wobei das basale Roboterarmende (4) mit der Roboterbasis (3) verbunden ist;
- eine erste Magnetquelle (7), die dazu konfiguriert ist, ein erstes Magnetfeld zu erzeugen, und mindestens eine zweite Magnetquelle (8), die dazu konfiguriert ist, ein zweites Magnetfeld zu erzeugen, wobei das erste Magnetfeld konstant ist und wobei das zweite Magnetfeld periodisch variabel ist,
wobei die erste Magnetquelle (7) mit dem distalen Roboterarmende (5) verbunden ist, wobei die erste Magnetquelle (7) integral mit dem distalen Roboterarmende (5) in einer Vielzahl von Betriebspositionen der ersten Magnetquelle in Bezug auf die Roboterbasis (3) bewegbar ist, um zumindest einen Einführabschnitt (102) der medizinischen Vorrichtung (101) durch Bewegen des Roboterarms (2) in das erste Magnetfeld einzutauchen;
**dadurch gekennzeichnet, dass**
die mindestens eine zweite Magnetquelle (8) an der Roboterbasis (3) so fixiert ist, dass sie für jede erste Magnetquellenposition der Magnetquelle (7) in einer zweiten Magnetquellenposition angeordnet ist, die in Bezug auf die Roboterbasis (3) fest und integral ist.

2. Magnetische Roboteranordnung (1) nach dem vorhergehenden Anspruch, wobei die mindestens eine zweite Magnetquelle (8) so ausgerichtet ist, dass, wenn sich die erste Magnetquelle (7) in einer beliebigen ersten Magnetquellen-Betriebsposition der Vielzahl von ersten Magnetquellen-Betriebspositionen befindet, das zweite Magnetfeld dem ersten Magnetfeld so überlagert wird, dass zumindest der Einführabschnitt (102) in das erste Magnetfeld und das zweite Magnetfeld eingetaucht wird, und/oder so, dass ein Parallelismus zwischen den Magnetfeldvektoren des zweiten Magnetfelds und den Magnetfeldvektoren des ersten Magnetfelds in einer beliebigen Position vermieden wird, in der das erste Magnetfeld dem zweiten Magnetfeld überlagert wird.

3. Magnetische Roboteranordnung (1) nach einem der vorhergehenden Ansprüche, die eines oder mehrere der folgenden Merkmale oder eine Kombination davon umfasst:
- wobei die magnetische Roboteranordnung (1) einen Effektor (6) umfasst, der direkt mit dem distalen Roboterarmende (5) verbunden ist, wobei der Effektor (6) die erste Magnetquelle (7) umfasst, wobei das Stützen weiterer Magnetquellen, die dazu konfiguriert sind, ein jeweiliges weiteres Magnetfeld zu erzeugen, vermieden wird;
und/oder wobei die erste Magnetquelle (7) ein Permanentmagnet (9) ist, der dazu konfiguriert ist, das erste Magnetfeld zu erzeugen.

4. Magnetische Roboteranordnung (1) nach einem der vorhergehenden Ansprüche, die eines oder mehrere der folgenden Merkmale oder eine Kombination davon umfasst:
- wobei die mindestens eine zweite Magnetquelle (8) mindestens eine Wicklung (10) umfasst, die dazu konfiguriert ist, das zweite Magnetfeld zu erzeugen, wenn sie von einem Strom durchflossen wird, und/oder
- wobei die mindestens eine Magnetquelle (8) ein Elektromagnet ist, der dazu konfiguriert ist, das zweite Magnetfeld zu erzeugen, und/oder
- wobei die Roboterbasis (3) einen Basiskörper (11) umfasst, der mindestens einen Sitz (12) der zweiten Magnetquelle begrenzt, wobei die mindestens eine zweite Magnetquelle (8) in dem Sitz (12) der zweiten Magnetquelle in Verbindung mit der Roboterbasis (3) untergebracht ist, und wobei die mindestens eine Magnetquelle (8) durch einen elektrischen Anschluss einer zweiten Magnetquelle elektrisch gespeist wird, wobei der elektrische Anschluss innerhalb des Basiskörpers (11) untergebracht ist.

5. Magnetische Roboteranordnung (1) nach dem vorhergehenden Anspruch, die eines oder mehrere der folgenden Merkmale oder eine Kombination davon umfasst:
- wobei die Roboterbasis (3) einen Stützabschnitt (13) umfasst, der geeignet ist, die mindestens eine zweite Magnetquelle (8) direkt zu stützen, wobei der Stützabschnitt (13) den mindestens einen Sitz (12) der zweiten Magnetquelle zumindest teilweise begrenzt; und/oder
- wobei die Roboterbasis (3) reversible Verbindungsmittel (13) umfasst, die dazu konfiguriert sind, die mindestens eine zweite Magnetquelle (8) reversibel mit der Roboterbasis (3) zu verbinden, was einen Austausch der mindestens einen zweiten Magnetquelle (8) durch eine andere aus einem Satz von zweiten Magnetquellen ermöglicht; und/oder
- wobei die mindestens eine zweite Magnetquelle (8) einen Spulenträger (21) umfasst, der geeignet ist, die mindestens eine Wicklung (10) zu stützen, wobei der Spulenträger (21) mindestens einen hohlen Abschnitt aufweist, um die mindestens eine Wicklung (10) elektrisch zu verbinden, und/oder
- wobei die mindestens eine zweite Magnetquelle (8) einen Magnetkern (25) umfasst, um die Linien des zweiten Magnetfelds im Material des Magnetkerns (25) zu konzentrieren.

6. Magnetische Roboteranordnung (1) nach einem der Ansprüche 4 bis 5, wobei die Roboterbasis (3) ein Bedienfeld (26) umfasst, das geeignet ist, die magnetische Roboteranordnung (1) zu steuern,
wobei die magnetische Roboteranordnung (1) eine Frontzone definiert, in der das distale Roboterarmende (5) hauptsächlich für eine Bewegung geeignet ist, wobei das Bedienfeld (26) am Basiskörper (11) in einer der Frontzone gegenüberliegenden Zone angeordnet ist,
wobei der Basiskörper (4) eine Abschirmung des zweiten Magnetfelds zumindest in Richtung des vorderen Teils der magnetischen Roboteranordnung (1) vermeidet,
und wobei der Basiskörper (11) das zweite Magnetfeld zumindest in Richtung des Bedienfelds abschirmt.

7. Magnetische Roboteranordnung (1) nach einem der Ansprüche 4 bis 6, wobei die mindestens eine Wicklung (10) eine Wicklungsachse (A) definiert, und wobei die Roboterbasis (3) einen Sockel (29) umfasst, der geeignet ist, auf einer Stützfläche oder einem Boden zu ruhen, um den Roboterarm (2) stabil zu stützen, wobei der Sockel (29) eine Anlagefläche umfasst, wobei die Wicklungsachse (A) parallel zur Anlagefläche verläuft oder wobei die Wicklungsachse (A) einen Wicklungswinkel zwischen 0 und 60 Grad mit der Anlagefläche des Sockels (29) bildet.

8. Magnetisches Robotersystem (100) zur magnetischen Lokalisierung einer medizinischen Vorrichtung (101), umfassend:
- eine magnetische Roboteranordnung (1) nach einem der vorhergehenden Ansprüche;
- die medizinische Vorrichtung (101), wobei die medizinische Vorrichtung (101) einen Einführabschnitt (102) umfasst, der geeignet ist, in einen Hohlraum eingeführt zu werden,
wobei der Einführabschnitt (102) eine Vielzahl von Magnetfeldsensoren (18) umfasst, die dazu konfiguriert sind, einen dreidimensionalen Magnetfeldvektor eines Magnetfelds zu erfassen, in das der Einführabschnitt (102) eingetaucht ist,
wobei der Einführabschnitt (102) einen dreiachsigen Beschleunigungsmesser (19) umfasst, der dazu konfiguriert ist, einen dreidimensionalen Vektor der dreidimensionalen Beschleunigung des Einführabschnitts (102) zu erfassen,
wobei der Einführabschnitt (102) ein dreiachsiges Gyroskop (20) umfasst, das dazu konfiguriert ist, mindestens drei Winkelgeschwindigkeiten des Einführabschnitts (102) in Bezug auf drei zueinander einfallende und/oder orthogonale Achsen zu erfassen;
- eine erste Magnetquellenposition-Steuereinheit (15), die dazu konfiguriert ist, die Position der ersten Magnetquelle zwischen der Vielzahl von ersten Magnetquellenpositionen der ersten Magnetquelle (7) zu erfassen,
- eine zweite Magnetfeld-Steuereinheit (14), die dazu konfiguriert ist, eine vordefinierte Frequenz und eine vordefinierte Wellenform zu steuern, um das zweite Magnetfeld mit der mindestens einen zweiten Magnetquelle (8) zu erzeugen;
- eine Verarbeitungseinheit (17), die dazu konfiguriert ist, ein Rollen und ein Nicken des Einführabschnitts (102) in Bezug auf einen Referenzpunkt der Roboterbasis (4) basierend auf dem dreidimensionalen Beschleunigungsvektor bzw. den mindestens drei Winkelgeschwindigkeiten, die durch den dreiachsigen Beschleunigungsmesser (19) und das dreiachsige Gyroskop (20) erfasst wurden, zu berechnen und/oder zu schätzen,
wobei die Verarbeitungseinheit (17) dazu konfiguriert ist, eine Position der medizinischen Vorrichtung (Xp, Yp, Zp) des Einführabschnitts (102) und ein Gieren des Einführabschnitts (102) in Bezug auf den Referenzpunkt der Roboterbasis (4) zu berechnen und/oder zu schätzen, und zwar basierend auf dem berechneten Rollen, dem berechneten Nicken, dem erfassten dreidimensionalen Magnetfeldvektor, einer relativen Position der Magnetquellen, die durch die Differenz zwischen der erfassten ersten Magnetquellenposition und der zweiten Magnetquellenposition definiert ist, die fest und integral mit der Roboterbasis (4) ist, einer relativen Position der zweiten Magnetquelle, die durch die Differenz zwischen der zweiten Magnetquellenposition, die fest und integral mit der Roboterbasis (4) ist, und dem Referenzpunkt der Roboterbasis (4) definiert ist, aus der vordefinierten Frequenz und der vordefinierten Wellenform.

9. Verfahren zur Lokalisierung einer medizinischen Vorrichtung (101), die in ein Magnetfeld eingetaucht ist, wobei die medizinische Vorrichtung (101) einen Einführabschnitt (102) umfasst, der geeignet ist, in einen Hohlraum eingeführt zu werden,
wobei der Einführabschnitt (102) eine Vielzahl von Magnetfeldsensoren (18) umfasst, die dazu konfiguriert sind, das Magnetfeld zu erfassen, in das der Einführabschnitt (102) eingetaucht ist,
einen dreiachsigen Beschleunigungsmesser (19), der dazu konfiguriert ist, einen dreidimensionalen Beschleunigungsvektor des Einführabschnitts (102) zu erfassen,
ein dreiachsiges Gyroskop (20), das dazu konfiguriert ist, mindestens drei Winkelgeschwindigkeiten des Einführabschnitts (102) in Bezug auf drei zueinander einfallende und/oder orthogonale Achsen zu erfassen;
wobei das Verfahren die Schritte umfasst:
- Bereitstellen einer Roboteranordnung (1) nach einem der Ansprüche 1 bis 7,
- Erzeugen des ersten Magnetfelds bzw. des zweiten Magnetfelds durch die erste Magnetquelle (7) und die zweite Magnetquelle (8), so dass die medizinische Vorrichtung (101) zumindest in die Überlagerung des ersten Magnetfelds und des zweiten Magnetfelds eingetaucht ist,
- Konstanthalten des ersten Magnetfelds,
- periodisches Variieren des zweiten Magnetfelds mit einer vordefinierten periodischen Wellenfunktion und einer vordefinierten Frequenz,
- Erfassen der Position der zweiten Magnetquelle, die fest und integral mit der Roboterbasis (4) ist,
- Definieren einer Referenzposition der Roboterbasis (4),
- Berechnen einer relativen Position der zweiten Magnetquelle, die durch die Differenz zwischen der zweiten Magnetquellenposition und der Referenzposition definiert ist,
- Erfassen, Ermitteln und Speichern der ersten Magnetquellenposition der ersten Magnetquelle (7),
- Berechnen einer relativen Position der Magnetquellen, die durch die Differenz zwischen der erfassten ersten Magnetquellenposition und der zweiten Magnetquellenposition, die fest und integral mit der Roboterbasis (4) ist, definiert ist,
- Erfassen, Ermitteln und Speichern des dreidimensionalen Magnetfeldvektors, des dreidimensionalen Beschleunigungsvektors und der mindestens drei Winkelgeschwindigkeiten mit einer Erfassungsfrequenz,
- Berechnen und Speichern eines Rollens und eines Nickens des Einführabschnitts (102) in Bezug auf einen Referenzpunkt der Roboterbasis (4) basierend auf dem dreidimensionalen Beschleunigungsvektor bzw. den mindestens drei Winkelgeschwindigkeiten, die durch den dreiachsigen Beschleunigungsmesser (19) und das dreiachsige Gyroskop (20) erfasst wurden,
- Berechnen einer Position der medizinischen Vorrichtung (Xp, Yp, Zp) des Einführabschnitts (102) und eines Gierens des Einführabschnitts (102) in Bezug auf den Referenzpunkt der Roboterbasis (4) basierend auf dem berechneten Rollen, dem berechneten Nicken, der berechneten relativen Position der Magnetquellen, der berechneten relativen Position der zweiten Magnetquelle, dem dreidimensionalen Magnetfeldvektor, der vordefinierten periodischen Wellenfunktion und der vordefinierten Frequenz.

10. Verfahren nach dem vorhergehenden Anspruch, wobei das Verfahren ferner die Schritte umfasst:
- Berechnen und Speichern, mit einer Berechnungsfrequenz, einer ersten Funktion, zum Beispiel einen Durchschnitt oder Median oder einen zuletzt erfassten Wert, der erfassten dreidimensionalen Beschleunigungsvektoren und/oder deren bei der Erfassungsfrequenz erfassten kartesischen Komponenten, einer zweiten Funktion, zum Beispiel einen Durchschnitt oder Median oder einen zuletzt erfassten Wert, der erfassten dreidimensionalen Winkelgeschwindigkeitsvektoren und/oder deren bei der Erfassungsfrequenz erfassten kartesischen Komponenten, und eines Durchschnitts der erfassten dreidimensionalen Magnetfeldvektoren und/oder deren bei der Erfassungsfrequenz erfassten kartesischen Komponenten, wobei der Durchschnitt des bei der Erfassungsfrequenz erfassten dreidimensionalen Magnetfeldvektors bezeichnend für das erfasste erste Magnetfeld ist,
- Berechnen und Speichern eines Durchschnitts der ersten Magnetquellenposition der ersten Magnetquelle (7) bei der Berechnungsfrequenz als Durchschnitt zwischen den erfassten ersten Magnetquellenpositionen,
- Berechnen und Speichern der Phase und Amplitude des erfassten Magnetfelds, das der zweiten Magnetfeldquelle (8) zugeordnet ist, bei der Berechnungsfrequenz basierend auf einer Sequenz von dreidimensionalen Magnetfeldvektoren, die von der Vielzahl von Magnetfeldsensoren (18) in einem Zeitintervall zwischen einer Amplituden- und Phasenberechnung und der nächsten erfasst wurden, wobei die bei der Berechnungsfrequenz berechnete Magnetfeldphase und -amplitude bezeichnend für das zweite Magnetfeld sind, wobei das Rollen und ein Nicken des Einführabschnitts (102) basierend auf der ersten Funktion des dreidimensionalen Beschleunigungsvektors und der zweiten Funktion der mindestens drei erfassten Winkelgeschwindigkeiten geschätzt werden;
wobei die Position der medizinischen Vorrichtung (Xp, Yp, Zp) und das Gieren basierend auf dem bei der Berechnungsfrequenz berechneten Rollen, dem bei der Berechnungsfrequenz berechneten Nicken, dem bei der Berechnungsfrequenz berechneten durchschnittlichen Magnetfeld, der berechneten Magnetfeldamplitude und -phase, dem Durchschnitt der ersten Magnetquellenposition sowie auf Modellen des von der ersten Magnetquelle (7) und der zweiten Magnetquelle (8) erzeugten Magnetfelds geschätzt werden.

11. Verfahren nach dem vorhergehenden Anspruch,
wobei die Erfassungsfrequenz mindestens eine Größenordnung, vorzugsweise zwei Größenordnungen, höher ist als die Berechnungsfrequenz.

## Revendications

1. Ensemble robotique magnétique (1) pour localiser magnétiquement un dispositif médical (101), comprenant :
- un bras robotisé (2),
- une base de robot (3) configurée pour supporter le bras robotisé (2), dans laquelle le bras robotisé (2) est mobile dans une pluralité de configurations par rapport à ladite base de robot (3), dans laquelle le bras robotisé (2) s'étend au moins entre une extrémité de base de bras robotisé (4) et une extrémité distale de bras robotisé (5), dans laquelle ladite extrémité de base de bras robotisé (4) est reliée à ladite base de robot (3) ;
- une première source magnétique (7) configurée pour générer un premier champ magnétique et au moins une deuxième source magnétique (8) configurée pour générer un deuxième champ magnétique, dans lequel le premier champ magnétique est constant et dans lequel le deuxième champ magnétique est périodiquement variable,
dans lequel la première source magnétique (7) est reliée à ladite extrémité distale de bras robotisé (5), dans lequel la première source magnétique (7) est mobile intégralement avec ladite extrémité distale de bras robotisé (5) dans une pluralité de positions de fonctionnement de la première source magnétique par rapport à ladite base de robot (3) de manière à immerger au moins une partie d'insertion (102) du dispositif médical (101) dans le premier champ magnétique en déplaçant ledit bras robotisé (2) ;
**caractérisé en ce que**
ladite au moins une deuxième source magnétique (8) est contrainte à ladite base de robot (3) de manière à être disposée dans une deuxième position de source magnétique qui est fixe et solidaire par rapport à ladite base de robot (3) pour chaque première position de source magnétique de ladite première source magnétique (7).

2. Ensemble robotique magnétique (1) selon la revendication précédente, dans lequel ladite au moins une deuxième source magnétique (8) est orientée de telle sorte que lorsque ladite première source magnétique (7) est dans n'importe quelle première position de fonctionnement de source magnétique de ladite pluralité de premières positions de fonctionnement de source magnétique, le deuxième champ magnétique est superposé au premier champ magnétique de manière à immerger au moins la partie d'insertion (102) dans le premier champ magnétique et le deuxième champ magnétique, et/ou de manière à éviter un parallélisme entre les vecteurs de champ magnétique dudit deuxième champ magnétique et les vecteurs de champ magnétique dudit premier champ magnétique dans n'importe quelle position dans laquelle le premier champ magnétique est superposé au deuxième champ magnétique.

3. Ensemble robotique magnétique (1) selon l'une quelconque des revendications précédentes, comprenant une ou plusieurs des caractéristiques suivantes ou une combinaison de celles-ci :
- dans lequel ledit ensemble robotique magnétique (1) comprend un effecteur (6) directement relié à ladite extrémité distale (5) du bras robotique, dans lequel ledit effecteur (6) comprend ladite première source magnétique (7) évitant de supporter d'autres sources magnétiques configurées pour générer un champ magnétique supplémentaire respectif ;
et/ou dans lequel ladite première source magnétique (7) est un aimant permanent (9) configuré pour générer ledit premier champ magnétique.

4. Ensemble robotique magnétique (1) selon l'une quelconque des revendications précédentes, comprenant une ou plusieurs des caractéristiques suivantes ou une combinaison de celles-ci :
- dans lequel ladite au moins une deuxième source magnétique (8) comprend au moins un enroulement (10) configuré pour générer ledit deuxième champ magnétique lorsqu'il est traversé par un courant, et/ou
- dans lequel ladite au moins une source magnétique (8) est un électroaimant configuré pour générer ledit deuxième champ magnétique, et/ou
- dans lequel ladite base de robot (3) comprend un corps de base (11) délimitant au moins un siège (12) d'une deuxième source magnétique, dans lequel ladite au moins une deuxième source magnétique (8) est logée dans ledit siège (12) de la deuxième source magnétique en connexion avec ladite base de robot (3), et dans lequel ladite au moins une source magnétique (8) est alimentée électriquement par une connexion électrique d'une deuxième source magnétique, dans lequel ladite connexion électrique est logée à l'intérieur dudit corps de base (11).

5. Ensemble robotique magnétique (1) selon la revendication précédente, comprenant une ou plusieurs des caractéristiques suivantes ou une combinaison de celles-ci :
- dans lequel ladite base de robot (3) comprend une partie de support (13) adaptée pour supporter directement ladite au moins une deuxième source magnétique (8), dans lequel ladite partie de support (13) délimite au moins partiellement ledit au moins un siège (12) de la deuxième source magnétique ; et/ou
- dans lequel ladite base de robot (3) comprend un moyen de liaison réversible (13) configuré pour relier de manière réversible ladite au moins une deuxième source magnétique (8) à ladite base de robot (3) permettant un remplacement de ladite au moins une deuxième source magnétique (8) par une autre provenant d'un ensemble de deuxièmes sources magnétiques ; et/ou
- dans lequel ladite au moins une deuxième source magnétique (8) comprend un support de bobine (21) adapté pour supporter ledit au moins un enroulement (10), dans lequel ledit support de bobine (21) a au moins une partie creuse pour connecter électriquement ledit au moins un enroulement (10), et/ou
- dans lequel la au moins une deuxième source magnétique (8) comprend un noyau magnétique (25) de manière à concentrer les lignes du deuxième champ magnétique dans le matériau du noyau magnétique (25).

6. Ensemble robotique magnétique (1) selon l'une quelconque des revendications 4 à 5, dans lequel ladite base de robot (3) comprend un panneau de commande (26) adapté pour commander ledit ensemble robotique magnétique (1),
dans lequel ledit ensemble robotique magnétique (1) définit une zone avant dans laquelle l'extrémité distale (5) du bras robotique est principalement adaptée pour se déplacer, dans lequel le panneau de commande (26) est disposé sur le corps de base (11) dans une zone opposée à la zone avant,
dans lequel ledit corps de base (4) évite de protéger le deuxième champ magnétique au moins vers ladite partie avant de l'ensemble robotique magnétique (1),
et dans lequel ledit corps de base (11) protège le deuxième champ magnétique au moins vers le panneau de commande.

7. Ensemble robotique magnétique (1) selon l'une quelconque des revendications 4 à 6, dans lequel ledit au moins un enroulement (10) définit un axe d'enroulement (A), et dans lequel ladite base de robot (3) comprend un socle (29) adapté pour reposer sur une surface de support ou un plancher pour supporter de manière stable le bras robotique (2), dans lequel le socle (29) comprend une surface de butée, dans lequel l'axe d'enroulement (A) est parallèle à la surface de butée ou dans lequel l'axe d'enroulement (A) forme un angle d'enroulement entre 0 et 60 degrés avec la surface de butée dudit socle (29).

8. Système robotique magnétique (100) pour localiser magnétiquement un dispositif médical (101), comprenant :
- un ensemble robotique magnétique (1) selon l'une quelconque des revendications précédentes ;
- ledit dispositif médical (101), dans lequel ledit dispositif médical (101) comprend une partie d'insertion (102) adaptée pour être introduite dans une cavité,
dans lequel ladite partie d'insertion (102) comprend une pluralité de capteurs de champ magnétique (18) configurés pour détecter un vecteur de champ magnétique tridimensionnel d'un champ magnétique dans lequel ladite partie d'insertion (102) est immergée,
dans lequel ladite partie d'insertion (102) comprend un accéléromètre triaxial (19) configuré pour détecter un vecteur tridimensionnel d'accélération tridimensionnelle de ladite partie d'insertion (102),
dans lequel ladite partie d'insertion (102) comprend un gyroscope triaxial (20) configuré pour détecter au moins trois vitesses angulaires de ladite partie d'insertion (102) par rapport à trois axes mutuellement incidents et/ou orthogonaux ;
- une première unité de commande de position de source magnétique (15) configurée pour détecter la position de la première source magnétique entre ladite pluralité de premières positions de source magnétique de la première source magnétique (7),
- une deuxième unité de commande de champ magnétique (14) configurée pour commander une fréquence prédéfinie et une forme d'onde prédéfinie pour générer ledit deuxième champ magnétique avec ladite au moins une deuxième source magnétique (8) ;
- une unité de traitement (17) configurée pour calculer et/ou estimer un roulis et un pas de ladite partie d'insertion (102) par rapport à un point de référence de ladite base de robot (4) sur la base dudit vecteur d'accélération tridimensionnel et desdites au moins trois vitesses angulaires détectées par ledit accéléromètre triaxial (19) et ledit gyroscope triaxial (20), respectivement,
dans lequel ladite unité de traitement (17) est configurée pour calculer et/ou estimer une position de dispositif médical (Xp, Yp, Zp) de ladite partie d'insertion (102) et un lacet de ladite partie d'insertion (102) par rapport audit point de référence de ladite base de robot (4) sur la base du roulis calculé, du pas calculé, du vecteur de champ magnétique tridimensionnel détecté, d'une position relative de sources magnétiques définie par la différence entre la première position de source magnétique détectée et la deuxième position de source magnétique fixe et solidaire de la base de robot (4), d'une position relative de deuxième source magnétique définie par la différence entre la deuxième position de source magnétique fixe et solidaire de la base de robot (4) et le point de référence de ladite base de robot (4), à partir de la fréquence prédéfinie et de la forme d'onde prédéfinie.

9. Procédé pour localiser un dispositif médical (101) immergé dans un champ magnétique, dans lequel ledit dispositif médical (101) comprend une partie d'insertion (102) adaptée pour être introduite dans une cavité,
dans lequel ladite partie d'insertion (102) comprend une pluralité de capteurs de champ magnétique (18) configurés pour détecter ledit champ magnétique dans lequel ladite partie d'insertion (102) est immergée,
un accéléromètre triaxial (19) configuré pour détecter un vecteur d'accélération tridimensionnel de ladite partie d'insertion (102),
un gyroscope triaxial (20) configuré pour détecter au moins trois vitesses angulaires de ladite partie d'insertion (102) par rapport à trois axes mutuellement incidents et/ou orthogonaux ;
dans lequel ledit procédé comprend les étapes consistant à :
- prévoir un ensemble robot (1) selon l'une quelconque des revendications 1 à 7,
- générer, par ladite première source magnétique (7) et laditedeuxième source magnétique (8), ledit premier champ magnétique et leditdeuxième champ magnétique, respectivement, de sorte que le dispositif médical (101) soit immergé au moins dans la superposition du premier champ magnétique et le deuxième champ magnétique,
- maintenir constant le premier champ magnétique,
- faire varier périodiquement le deuxième champ magnétique avec une fonction d'onde périodique prédéfinie et une fréquence prédéfinie,
- détecter la position de ladeuxième source magnétique fixe et solidaire de la base du robot (4),
- définir une position de référence de ladite base de robot (4),
- calculer une position relative de ladeuxième source magnétique définie par la différence entre ladeuxième position de la source magnétique et la position de référence,
- détecter, acquérir et stocker la première position de source magnétique de la première source magnétique (7),
- calculer une position relative des sources magnétiques définie par la différence entre la première position de source magnétique détectée et la deuxième position de source magnétique fixe et solidaire de la base du robot (4),
- détecter, acquérir et stocker à une fréquence d'acquisition ledit vecteur de champ magnétique tridimensionnel, ledit vecteur d'accélération tridimensionnel et lesdites au moins trois vitesses angulaires,
- calculer et stocker un roulis et un pas de ladite partie d'insertion (102) par rapport à un point de référence de ladite base de robot (4) sur la base dudit vecteur d'accélération tridimensionnel et desdites au moins trois vitesses angulaires détectées par ledit accéléromètre triaxial (19) et ledit gyroscope triaxial (20), respectivement,
- calculer une position de dispositif médical (Xp, Yp, Zp) de ladite partie d'insertion (102) et un lacet de ladite partie d'insertion (102) par rapport audit point de référence de ladite base de robot (4) sur la base du roulis calculé, du pas calculé, de la position relative calculée des sources magnétiques, de la position relative calculée de la deuxième source magnétique, du vecteur de champ magnétique tridimensionnel, de la fonction d'onde périodique prédéfinie et de la fréquence prédéfinie.

10. Procédé selon la revendication précédente, comprenant les étapes supplémentaires consistant à :
- calculer et stocker à une fréquence de calcul une première fonction, par exemple une moyenne ou médiane ou dernière valeur détectée, des vecteurs d'accélération tridimensionnels détectés et/ou de leurs composantes cartésiennes détectées à ladite fréquence d'acquisition, une deuxième fonction, par exemple une moyenne ou médiane ou dernière valeur détectée, des vecteurs de vitesse angulaire tridimensionnels détectés et/ou de leurs composantes cartésiennes détectées à ladite fréquence d'acquisition, et une moyenne des vecteurs de champ magnétique tridimensionnels détectés et/ou de leurs composantes cartésiennes détectées à ladite fréquence d'acquisition, dans lequel la moyenne du vecteur de champ magnétique tridimensionnel détecté à ladite fréquence d'acquisition est significative du premier champ magnétique détecté,
- calculer et stocker à ladite fréquence de calcul une moyenne de la première position de source magnétique de la première source magnétique (7) en tant que moyenne entre les premières positions de source magnétique détectées,
- calculer et stocker à ladite fréquence de calcul la phase et l'amplitude du champ magnétique détecté associé à la deuxième source de champ magnétique (8) sur la base d'une séquence de vecteurs de champ magnétique tridimensionnels détectés par la pluralité de capteurs de champ magnétique (18) dans un intervalle de temps entre un calcul d'amplitude et de phase et le suivant, dans lequel la phase et l'amplitude du champ magnétique calculées à la fréquence de calcul sont significatives du deuxième champ magnétique, dans lequel ledit roulis et un pas de ladite partie d'insertion (102) sont estimés sur la base de la première fonction du vecteur d'accélération tridimensionnel et de la deuxième fonction desdites au moins trois vitesses angulaires détectées ;
dans lequel ladite position du dispositif médical (Xp, Yp, Zp) et ledit lacet sont estimés sur la base dudit roulis calculé à ladite fréquence de calcul, dudit pas calculé à ladite fréquence de calcul, dudit champ magnétique moyen calculé à ladite fréquence de calcul, de l'amplitude et de la phase du champ magnétique calculées, de la moyenne de la première position de la source magnétique, sur des modèles de champ magnétique générés par ladite première source magnétique (7) et ladite deuxième source magnétique (8).

11. Procédé selon la revendication précédente,
dans lequel ladite fréquence d'acquisition est d'au moins un ordre de grandeur, de préférence de deux ordres de grandeur, supérieure à ladite fréquence de calcul.
